# EUROPÄISCHE PATENTSCHRIFT

(11) **EP 3 842 411 B1**
(45) Veröffentlichungstag und Bekanntmachung des Hinweises auf die Patenterteilung: **31.05.2023**
(21) Anmeldenummer: 20193080.7
(22) Anmeldetag: 17.12.2019
(51) Int. Cl.: C07C 69/608

(54) **3,3',3''-(CYCLOHEXAN-1,2,4-TRIYL)TRIPROPIONSÄURETRIMETHYLESTER**
3,3',3''-(CYCLOHEXANE-1,2,4-TRIYL)TRIPROPIONIC ACID TRIMETHYL ESTER
TRIMÉTHYLESTER ACIDE 3,3',3''-(CYCLOHEXANE-1,2,4-TRIYL)TRIPROPIONIQUE

(43) Veröffentlichungstag der Anmeldung: 30.06.2021
(62) Teilanmeldung aus: 19216884.7
(73) Patentinhaber: Evonik Operations GmbH, 45128 Essen (DE)
(72) Erfinder: Dong, Kaiwu, Bo Zhou, 236800 (CN); Franke, Robert, 45772 Marl (DE); Jackstell, Ralf, 18106 Rostock (DE); Beller, Matthias, 18211 Ostseebad Nienhagen (DE)
(74) Vertreter: Evonik Patent Association

(56) Entgegenhaltungen:
- EP-A2- 0 495 175

## Beschreibung

Die vorliegende Erfindung betrifft 3,3',3"-(Cyclohexan-1,2,4-triyl)tripropionsäuretrimethylester.

Die Alkoxycarbonylierung ethylenisch ungesättigter Verbindungen ist ein Prozess mit steigender Bedeutung. Unter einer Alkoxycarbonylierung versteht man die Umsetzung von ethylenisch ungesättigten Verbindungen, wie Olefinen, mit Kohlenmonoxid und Alkoholen in Gegenwart eines Metalls bzw. eines Metallkomplexes und eines Liganden zu den entsprechenden Estern:

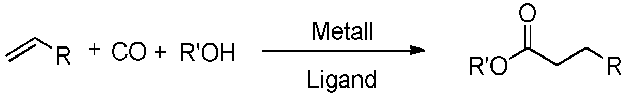

Mit einem derartigen Verfahren lassen sich alle drei Vinylgruppen von Trivinylcyclohexan in Ester umwandeln.

EP0 495 175 A2 beschreibt die Hydrocarboxyalkylierung von 1,2,4-Trivinylcyclohexan mit C1-C10-Alkoholen in Gegenwart eines Kobaltkatalysators, wobei Mischungen der entsprechenden Ester, die Verwendung als Monomere und Co-Monomere finden, erhalten werden.

Der vorliegenden Erfindung lag die Aufgabe zu Grunde, einen schnell gelierenden Weichmacher für Kunststoffe, insbesondere für PVC, bereitzustellen, welcher eine niedrige Verarbeitungstemperatur ermöglicht.

Diese Aufgabe wird gelöst durch eine Verbindung gemäß der Formel (1):

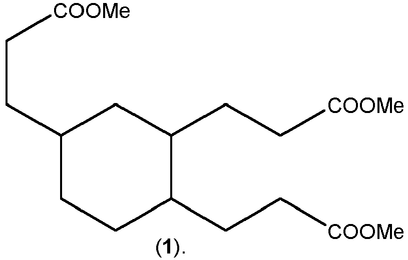

Vorzugsweise wird die Verbindung nach dem hier beschriebenen Verfahren hergestellt.

Im Folgenden soll die Erfindung anhand eines Ausführungsbeispiels näher erläutert werden.

### Synthese 3,3',3"-(Cyclohexan-1,2,4-triyl)tripropionsäuretrimethylester (1) ("Triester")

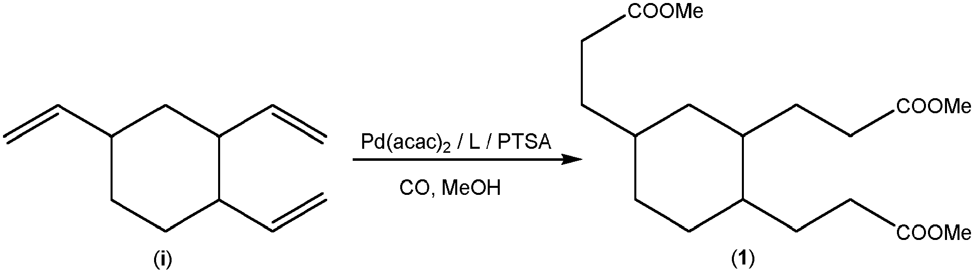

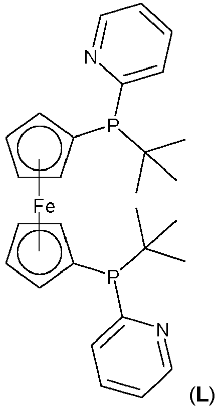

[Pd(acac)₂] (15,2 mg, 0,1 mol%), L (103 mg, 0,4 mol%) und p-Toluolsulfonsäure (PTSA) (143 mg, 1,5 mol%) wurden unter einer Argonatmosphäre in einen 100-ml-Stahlautoklaven gegeben. Dann wurden MeOH (30 ml) und Trivinylcyclohexan (i) (8,1 g, 50 mmol) mittels einer Spritze injiziert. Der Autoklav wurde dreimal mit CO gespült und anschließend mit einem CO-Druck von 40 bar beaufschlagt. Die Reaktion erfolgte über 10 h bei 110 °C. Danach wurde der Autoklav auf Raumtemperatur abgekühlt und entspannt. Das gewünschte Produkt wurde durch Destillation (165 °C bei 10⁻³ bar) aufgereinigt und mit ¹H-, ¹³C-NMR und HR-MS charakterisiert (15,6 g, 91% Ausbeute).

¹H-NMR (300 MHz, C₆D₆) δ = 3,39-3,37 (m, 9H), 2,24-1,86 (m, 7H), 1,48-0,27 (m, 14 H). ¹³C-NMR (75 MHz, C₆D₆) δ = 173,68-173,54 (m), 51,04, 40,60-40,47 (m), 38,24, 38,14, 37,51, 37,07, 36,54, 36,10, 35,52, 35,14, 33,87, 32,70, 32,55, 32,51, 32,38, 32,29, 32,23, 32,08, 31,97, 31,86, 31,76, 31,68, 31,63, 31,43, 30,98, 30,79, 30,75, 29,31, 28,52, 28,47, 28,34, 28,13, 28,11, 27,13, 26,58, 25,12, 20,79, 19,74.

MS (EI): 311 (13,40), 293 (3,65), 269 (75,76), 237 (60,40), 219 (25,13), 205 (100), 191 (17,62), 177 (14,83), 145 (24,59).

HR-MS (ESI): Berechnet C₁₈H₃₀O₆ [M + H]⁺: 343,21152, Gefunden: 343,21113.

### Synthese 3,3',3"-(Cyclohexan-1,2,4-triyl)tris(propan-1-ol) (2) ("Triol") (nicht erfindungsgemäß)

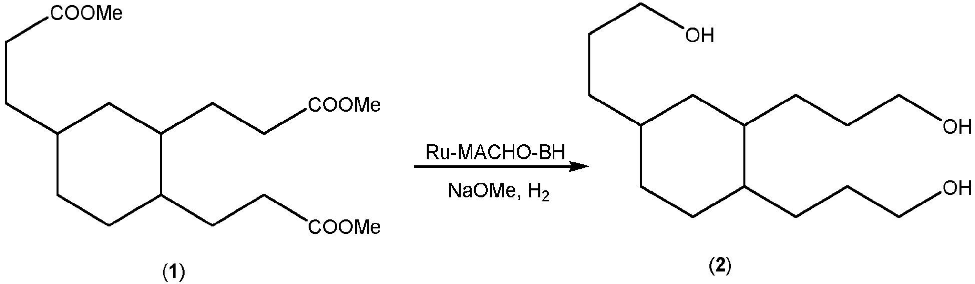

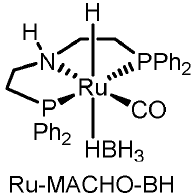

Ru-MACHO-BH (59 mg, 2 mol%) und NaOMe (27 mg, 10 mol%) wurden unter einer Argonatmosphäre in einen 25 ml-Stahlautoklaven gegeben. Dann wurden MeOH (8 ml) und der Triester (1) (1,93 g, 5,67 mmol) mittels Spritze injiziert. Der Autoklav wurde dreimal mit H₂ gespült und anschließend mit einem H₂-Druck von 50 bar beaufschlagt. Die Reaktion erfolgte über 10 h bei 100 °C. Der Autoklav wurde danach auf Raumtemperatur abgekühlt und entspannt. Das gewünschte Produkt wurde durch Filtration über Kieselgel aufgereinigt und durch ¹H-, ¹³C-NMR und HR-MS charakterisiert (1,4 g, 96% Ausbeute). ¹H-NMR (400 MHz, CD₃OD) δ = 3,32-3,30 (m, 6H), 1,85-1,77 (m, 2H), 1,63-0,62 (m, 19H). ¹³C-NMR (100 MHz, CD₃OD) δ = 63,53-63,29 (m), 42,69, 42,53, 42,39, 42,31, 40,34, 40,24, 39,28, 38,89, 38,12, 38,02, 37,58, 37,27, 35,89, 34,78, 34,72, 34,53, 34,37, 33,17, 31,52, 31,50, 31,40, 31,38, 31,05, 30,60, 30,48, 30,41, 30,32, 30,24, 30,17, 28,77, 28,22, 26,61, 22,53, 21,45. MS (EI): 222 (1,06), 194 (1,14), 181 (2,96), 163 (11,54), 135 (9,81), 121 (17,04), 107 (15,25), 93 (32,69).

HR-MS (ESI): Berechnet C₁₅H₃₀O₃ [M + H]⁺: 259,22677, Gefunden: 259,2269.

### Herstellung von Plastisolen

Es wurden PVC-Plastisole hergestellt, wie sie beispielsweise zur Fertigung von Deckstrichfilmen für Fußbodenbeläge verwendet werden. Die Angaben in der Plastisolrezeptur ist jeweils in Massenanteilen. Die Rezeptur der Polymerzusammensetzung ist in Tabelle 1 aufgelistet.

**Tabelle 1: Plastisolrezeptur**

| | phr |
|---|---|
| PVC (Vestolit B 7021 - Ultra; Fa. Vestolit) | 100 |
| Weichmacher | 50 |
| epoxidiertes Sojabohnenöl als Co-Stabilisator | 3 |
| (Drapex 39, Fa. Galata) | |
| Thermostabilisator auf Ca/Zn-Basis | 2 |
| (Reagens CLX/759/6PF) | |

| | |
|---|---|
| Angaben in phr (phr = parts per hundred parts resin) | |

Zuerst werden die flüssigen und dann die pulverförmigen Bestandteile in einen PE-Becher eingewogen. Von Hand wird die Mischung mit einem Salbenspatel so eingerührt, dass kein unbenetztes Pulver mehr vorhanden ist. Der Mischbecher wird dann in die Klemmvorrichtung eines Dissolverrührers eingespannt. Nach Einschalten des Rührers wird die Drehzahl langsam auf ca. 2000 UpM erhöht. Währenddessen wird das Plastisol vorsichtig entlüftet, der Druck muss 20 mbar unterschreiten.

Sobald das Plastisol eine Temperatur von ca. 30 °C erreicht hat, wird die Drehzahl auf ca. 350 UpM gesenkt. Ab jetzt wird das Plastisol für 9 Minuten bei dieser Drehzahl und einem Druck unterhalb von 20 mbar entlüftet. Damit war sichergestellt, dass die Homogenisierung des Plastisols bei einem definierten Energieeintrag erreicht wurde. Danach wird das Plastisol sofort für weitere Untersuchungen in einem Klimaschrank auf 25,0 °C temperiert.

### Gelierverhalten der Plastisole

Die Untersuchung des Gelierverhaltens der Pasten wurde mit einem Physica MCR 101 im Oszillationsmodus mit einem Platte-Platte Messsystem (PP25), welches schubspannungsgesteuert betrieben wurde, vorgenommen. Eine zusätzliche Temperierhaube wurde an das Gerät angeschlossen, um eine homogene Wärmeverteilung und eine gleichmäßige Probentemperatur zu erreichen.

Folgende Parameter wurden eingestellt:

| Modus: | Temperatur-Gradient |
|---|---|
| Start-Temperatur: | 25 °C |
| End-Temperatur: | 180 °C |
| Heiz/Kühlrate: | 5 °C/min |
| Oszillations-Frequenz: | 4-0,1 Hz Rampe logarithmisch |
| Kreisfrequenz Omega: | 10 1/s |
| Anzahl Messpunkte: | 63 |
| Messpunktdauer: | 0,5 min |
| Automatische Spaltnachführung F: | 0 N |
| Konstante Messpunktdauer | |
| Spaltweite | 0,5 mm |

### Durchführung der Messung

Auf die untere Messsystemplatte werden mit dem Spatel einige Gramm der zu messenden Paste luftblasenfrei aufgetragen. Dabei wird darauf geachtet, dass nach dem Zusammenfahren des Messsystems etwas Paste gleichmäßig aus dem Messsystem herausquellen kann (nicht mehr als 6 mm rundum). Anschließend wird die Temperierhaube über der Probe positioniert und die Messung gestartet. Bestimmt wird die komplexe Viskosität der Paste nach 24 Stunden (Lagerung der Paste bei 25 °C in einem Temperierschrank der Fa. Memmert) in Abhängigkeit von der Temperatur.

Als Maß für die Gelierung wird ein deutlicher Anstieg der komplexen Viskosität betrachtet. Als Vergleichswert wird daher die Temperatur bei Erreichen einer Pastenviskosität von 1000 Pas verwendet.

Der Versuche wurde mit drei Vergleichsplastisolen wiederholt, bei welchen jeweils ein anderer Weichmacher verwendet wurde.

**Tabelle 2: Gelierung der Plastisole nach 24 Stunden, Temperatur in °C bei Erreichen einer Pastenviskosität von 10³ Pa·s**

| Versuch | Weichmacher | Geliertemperatur [°C] |
|---|---|---|
| 1* | 3,3',3"-(Cyclohexan-1,2,4-triyl)tripropionsäuretrimethylester (**1**) | 65 |
| 2 | Diisononylphthalat (DINP), VESTINOL 9 von Evonik Performance Materials GmbH | 83 |
| 3 | 1,2-Cyclohexandicarbonsäurediisononylester (DINCH), ELATUR CH von Evonik Performance Materials GmbH | 101 |
| 4 | Diisopentylterephthalat (DPT), ELATUR DPT von Evonik Performance Materials GmbH | 70 |

| | | |
|---|---|---|
| * Versuch mit erfindungsgemäßer Verbindung | | |

Der angestrebte Wert von 1000 Pa*s konnte mit der erfindungsgemäßen Verbindung (1) bereits bei 65 °C erreicht werden. Solch niedrige Geliertemperaturen sind vorteilhaft für den Verarbeitungsprozess. Sie ermöglichen eine Plastisolverarbeitung bei niedrigeren Temperaturen.

## Patentansprüche

1. Verbindung gemäß der Formel (1):

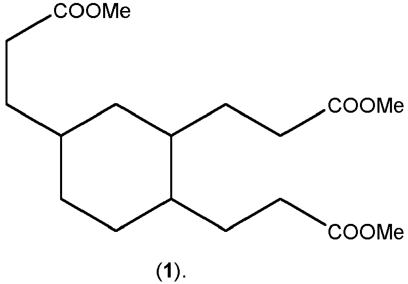

## Claims

1. Compound according to the formula (1):

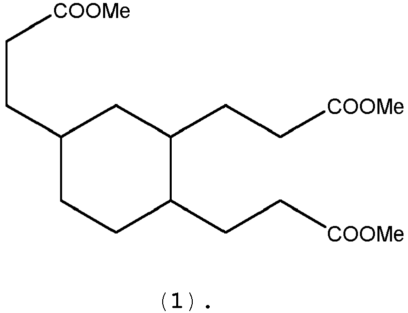

## Revendications

1. Composé selon la formule (1) :
